# EUROPEAN PATENT SPECIFICATION

(11) **EP 3 059 220 B1**
(45) Date of publication and mention of the grant of the patent: **05.12.2018**
(21) Application number: 13895696.6
(22) Date of filing: 18.10.2013
(51) Int. Cl.: C07C 51/363, C07C 63/70

(54) **METHOD OF PREPARING AND RECOVERING 2-METHYL-5-IODOBENZOIC ACID**
VERFAHREN ZUR HERSTELLUNG UND RÜCKGEWINNUNG VON 2-METHYL-5-IODOBENZOESÄURE
PROCÉDÉ DE PRÉPARATION ET DE RÉCUPÉRATION D'ACIDE 2-MÉTHYL-5-IODOBENZOIQUE

(43) Date of publication of application: 24.08.2016
(73) Proprietor: ABA Chemicals Corporation, Taicang, Jiangsu 215433 (CN)
(72) Inventor: LIN, Zhigang, Taicang Jiangsu 215433 (CN); LI, Hang, Taicang Jiangsu 215433 (CN); XU, Jun, Taicang Jiangsu 215433 (CN); QUE, Limin, Taicang Jiangsu 215433 (CN); QIN, Dongguang, Taicang Jiangsu 215433 (CN); JIANG, Yueheng, Taicang Jiangsu 215433 (CN)
(74) Representative: Gunzelmann, Rainer
(86) International application number: PCT/CN2013/001270
(87) International publication number: WO 2015/054806

(56) References cited:
- EP-A1- 1 642 881
- CN-A- 103 539 662
- IN-A1- 2307M UM2 010
- JP-A- H07 258 152

## Description

### TECHNICAL FIELD

The present invention relates to a method of preparing and recovering 2-methyl-5-iodobenzoic acid.

### BACKGROUND

2-methyl-5-iodobenzoic acid is an important intermediate of some new diabetes medications, SGLT2-type medications, so studying its preparation methods which are suitable for industrial production is of great significance.

Synthetic methods thereof reported in the literatures can be divided into two schemes:

### (1) direct iodination method

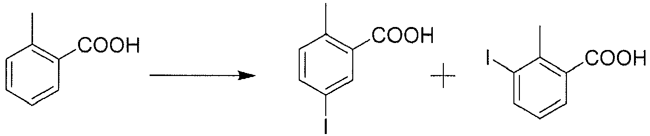

The main literatures are Indian Pat. Appl. 2010MU02307; WO2010022355; JP4332702; JMC, 52(16), 5228∼5240, 2009; WO2005003073 and so on. The main problem of such method is the presence of a large number of 3-iodo impurities, which result in considerable refinement loss.

### (2) diazotization-iodination method

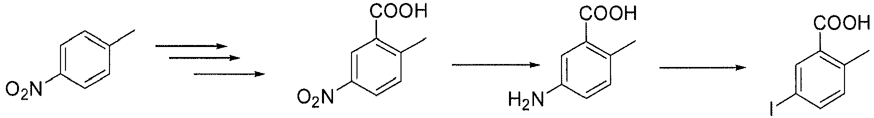

The main literatures are Bioorganic & Medicinal Chemistry Letter, 18(23): 6273-8, 2008; WO2006177669 and so on. This method has long reaction process and tedious treatment process, and is not suitable for industrial production.

### SUMMARRY

The object of the present invention is to provide a method of preparing and recovering 2-methyl-5-iodobenzoic acid, which mainly comprising the steps of:
1) utilizing o-methylbenzoic acid of formula I as raw material and iodinating via iodine/potassium persulfate in a mixed acid solvent to obtain a mixture of 2-methyl-5-iodobenzoic acid of formula II A, 2-methyl-3-iodobenzoic acid of formula II B and 2-methyl-3,5-diiodobenzoic acid of formula II C; 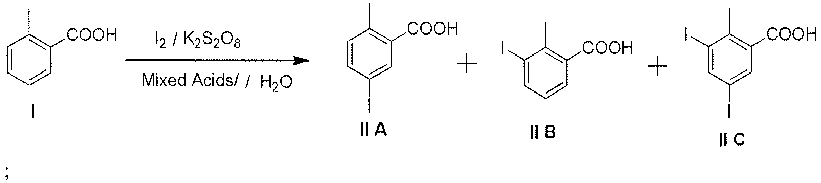
2) refining the mixture obtained in step 1) through recrystallization to obtain 2-methyl-5-iodobenzoic acid of formula II A and a recoverable mother liquor;
3) treating the recoverable mother liquor in a base/solvent system to obtain o-methylbenzoic acid of formula I and iodide ions, and then oxidating the iodide ions to recover iodine and putting the o-methylbenzoic acid of formula I into the reaction for reuse.

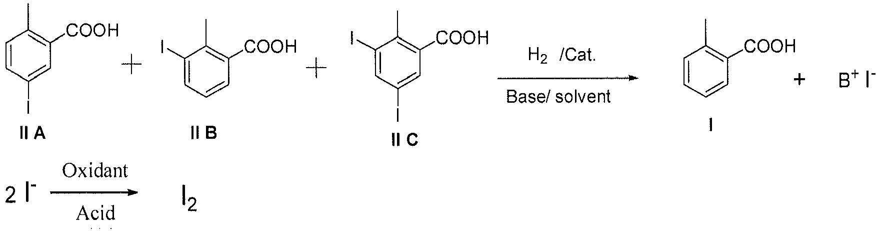

Wherein, the mixed acid in step 1) is a mixed solution of more than two of acetic acid, propionic acid, butyric acid and sulfuric acid in any proportion.

Wherein, step 2) comprises refining the mixture in a mixed solvent of acid/water to obtain 2-methyl-5-iodobenzoic acid. Preferably, the acid is any one of acetic acid, propionic acid, butyric acid and sulfuric acid. Wherein, the weight ratio of the acid/water is 1/0.01∼2, preferably 1/0.1∼1.

Wherein, the recoverable mother liquor in step 2) is a mixture of o-methylbenzoic acid, 2-methyl-5-iodobenzoic acid, 2-methyl-3-iodobenzoic acid and 2-methyl-3,5-diiodobenzoic acid.

Wherein, the refining process in step 2) comprises: adding 70% acetic acid/water into the mixture obtained in step 1), heating up until the solution become clear, slowly cooling to 15∼20 °C, suction filtering, washing the solid with a small amount of 50% acetic acid aqueous and drying to get a white product, i.e., the 2-methyl-5-iodobenzoic acid of formula II A.

Wherein, the base in step 3) is lithium hydroxide, sodium hydroxide or potassium hydroxide.

Wherein, the solvent in step 3) is water, or a water-miscible organic solvent. Preferably, the organic solvent is alcoholic solvent or ether solvent.

Wherein the alcoholic solvent is methanol or ethanol, and the ether solvent is THF.

The above-described preparation method has inexpensive and readily available raw materials; is easy of preparation operation, has low equipment requirements, and is suitable for large-scale industrial production.

To make above and other objects, features and advantages of the present invention more clear and comprehensible, preferred embodiments are described in detail below.

### DETAILED DESCRIPTION

### Example 1

### Synthesis of 2-methyl-5-iodobenzoic acid

The reaction was conducted in a 500 ml reaction flask and under nitrogen atmosphere, adding acetic acid (200 g), water (20 g), concentrated sulfuric acid (20 g) at room temperature and mixing evenly by stirring, then successively adding o-methylbenzoic acid (49 g, 0.36 mol), potassium persulfate (50 g), and finally iodine (38 g, 0.15 mol); then raising the temperature of the solution slightly; after stirring for 30 minutes, slowly raising to 50 °C and incubating for 1 hour; then slowly raising to 70 °C and incubating for 2 hours; and then slowly raising to 90 °C and incubating 3-4 hours; the color of the iodine in the reaction liquid gradually disappeared and the reaction liquid presented light yellow. The reaction liquid was sampled for HPLC analysis (o-methylbenzoic acid ∼ 10%; 2-methyl-5-iodobenzoic acid ∼ 75%; 2-methyl-3-iodobenzoic acid∼15%; 2- methyl-3, 5-diiodobenzoic acid < 1%).

Stop the reaction, slowly cooling the reaction liquid to 15∼20 °C, suction filtering, washing and drying to get a crude product∼55g (the yield was 70%, calculated by iodine); HPLC: 2- methyl-5-iodobenzoic acid∼96%; 2-methyl-3-iodobenzoic acid∼3%; 2-methyl benzoic acid ∼0.5%; the mother liquor was a mixture of o-methylbenzoic acid; 2-methyl-5-iodobenzoic acid; 2-methyl-3-iodobenzoic acid and 2-methyl-3,5-diiodobenzoic acid.

Refining: 200g of 70% acetic acid/water were added into 50g of the crude product for recrystallization refining: heating up until the solution became clear, slowly cooling to 15∼20 °C, suction filtering, washing the solid with a small amount of 50% acetic acid aqueous and drying to get a white product: 45g; the recrystallization yield was about 90%; HPLC of the product: 2-methyl-5-iodobenzoic acid > 99.5%; 2-methyl-3-iodobenzoic acid < 0.2%; 2-methyl benzoic acid < 0.1%. The mother liquor was a mixture of o-methylbenzoic acid; 2-methyl-5-iodobenzoic acid; 2-methyl-3-iodobenzoic acid and 2-methyl-3,5-diiodobenzoic acid.

### Recovery processing of 2-methyl-5-iodobenzoic acid mother liquor

Combine all the mother liquor, concentrating under vacuum, and recovering the acetic acid. After the recovery of the acetic acid was completed, the solution presented pasty, and a large amount of solid were precipitated after 100 g of water was added; then cooling to 0°C, filtering, and washing with water to get a recovery product of ∼40 g (The total recovery rate was ∼95%).

Add water (200 g) and 10% sodium hydroxide aqueous solution (100 ml) into the recoverable mother liquor (40 g), stirring until the solution became clear, adding Raney-Ni (4 g), and catalytic hydrogenating at a reaction temperature of 60-70 °C and under a reaction pressure of 3.0 Mpa for 6-8 hours, until the autoclave no longer absorb hydrogen, monitoring the completion of the reaction by HPLC, iodide < 0.1%. Stop the reaction, filtrating and recovering the catalysts. Add 30% hydrochloric acid into the filtrate until the filtrate was acidified to pH < 2, then precipitating a solid, cooling to 0 °C, filtering, and recovering 2-methyl benzoic acid (∼23 g) for refuse.

Slowly add 30% hydrogen peroxide (25 ml) dropwise into the aqueous phase in order to oxidize, stirring at room temperature overnight, precipitating iodine (∼15 g), cooling to 0°C, and recovering for refuse.

### Example 2

### Synthesis of 2-methyl-5-iodobenzoic acid

The reaction was conducted in a 500 ml reaction flask and under nitrogen atmosphere, adding propionic acid (200 g) and water (20 g) at room temperature and mixing evenly by stirring; then successively adding concentrated sulfuric acid (20 g), o-methylbenzoic acid (49 g, 0.36 mol), potassium persulfate (50 g), and finally iodine (38 g, 0.15 mol); then slightly raising the temperature of the solution; after stirring for 30 minutes, slowly raising to 50 °C and incubating for 1 hour; then slowly raising to 70 °C and incubating for 2 hours; and then slowly raising to 90 °C and incubating 3-4 hours; the color of the iodine in the reaction liquid gradually disappeared and the reaction liquid presented light yellow. The reaction liquid was sampled for HPLC analysis (o-methylbenzoic acid ∼ 8%; 2-methyl-5-iodobenzoic acid ∼ 80%; 2-methyl-3-iodobenzoic acid ∼11%; 2- methyl-3, 5-diiodobenzoic acid < 1%).

Stop the reaction, slowly cooling the reaction liquid to 15∼20 °C, filtering, washing and drying to get a crude product ∼58.9 g (the yield was 70%, calculated by iodine); HPLC: 2-methyl-5-iodobenzoic acid ∼ 97%; 2-methyl-3-iodobenzoic acid ∼2%; 2-methyl benzoic acid ∼0.5%; the mother liquor was a mixture of o-methylbenzoic acid, 2-methyl-5-iodobenzoic acid, 2-methyl-3-iodobenzoic acid and 2-methyl-3,5-diiodobenzoic acid.

Refining: 200 g of 70% propionic acid/water were added into 50 g of the crude product for recrystallization refining: heating up until the solution became clear, slowly cooling to 15∼20 °C, suction filtering, washing the solid with a small amount of 50% propionic acid aqueous and drying to get a white product: 45 g; the recrystallization yield was about 90%; HPLC of the product: 2-methyl-5-iodobenzoic acid > 99.5%; 2-methyl-3-iodobenzoic acid < 0.2%; 2-methyl benzoic acid < 0.1%. The mother liquor was a mixture of o-methylbenzoic acid, 2-methyl-5-iodobenzoic acid, 2-methyl-3-iodobenzoic acid and 2-methyl-3,5-diiodobenzoic acid.

### Recovery processing of 2-methyl-5-iodobenzoic acid mother liquor

Add water (200 g) and 10% sodium hydroxide aqueous solution (100 ml) into the recoverable mother liquor (40 g), stirring until the solution became clear, adding palladium on carbon (2 g), and catalytic hydrogenating at a reaction temperature of 30-40 °C and under a reaction pressure of 1.5 Mpa for 8-10 hours, until the autoclave no longer absorb hydrogen, monitoring the completion of the reaction by HPLC, iodide < 0.1%. Stop the reaction, filtrating and recovering the catalysts. Add 30% hydrochloric acid into the filtrate until the filtrate was acidified to pH < 2, then precipitating a solid, cooling to 0 °C, filtering, and recovering 2-methyl benzoic acid (∼23 g) for refuse.

Slowly add 30% hydrogen peroxide (25 ml) dropwise into the aqueous phase in order to oxidize, stirring at room temperature overnight, precipitating iodine (∼15 g), cooling to 0 °C, and recovering for refuse.

## Claims

1. A method of preparing 2-methyl-5-iodobenzoic acid, comprising the steps of:
1) utilizing o-methylbenzoic acid of formula I as raw material and iodinating via iodine/potassium persulfate in a mixed acid solvent to obtain a mixture of 2-methyl-5-iodobenzoic acid of formula II A, 2-methyl-3-iodobenzoic acid of formula II B and 2-methyl-3,5-diiodobenzoic acid of formula II C; 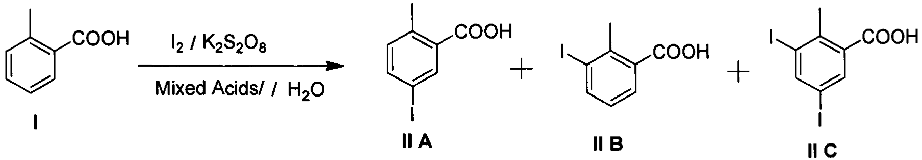
2) refining the mixture obtained in step 1) through recrystallization to obtain 2-methyl-5-iodobenzoic acid of formula II A and recoverable mother liquor;
3) treating and catalytically hydrogenating the recoverable mother liquor in a base/solvent system to obtain o-methylbenzoic acid of formula I and iodide ions, and then oxidating the iodide ions in the presence of an acid to recover iodine and putting the o-methylbenzoic acid of formula I into the reaction for reuse.

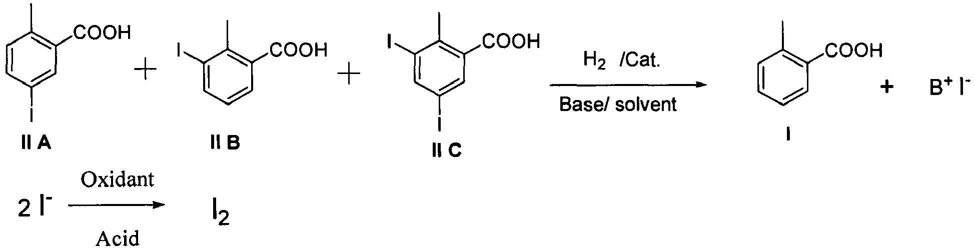

2. The method as claimed in claim 1, **characterized in that**, the mixed acid in step 1) is a mixed solution of more than two of acetic acid, propionic acid, butyric acid and sulfuric acid in any proportion.

3. The method as claimed in claim 1, **characterized in that**, step 2) comprises refining the mixture in a mixed solvent of acid/water to obtain 2-methyl-5-iodobenzoic acid.

4. The method as claimed in claim 3, **characterized in that**, the acid is any one of acetic acid, propionic acid, butyric acid and sulfuric acid.

5. The method as claimed in claim 1, **characterized in that**, the recoverable mother liquor in step 2) is a mixture of o-methylbenzoic acid, 2-methyl-5-iodobenzoic acid, 2-methyl-3-iodobenzoic acid and 2-methyl-3,5-diiodobenzoic acid.

6. The method as claimed in claim 1, **characterized in that**, the refining in step 2) comprises: adding 70% acetic acid/water into the mixture obtained in step 1), heating up until the solution become clear, slowly cooling to 15∼20°C, suction filtering, washing the solid with a small amount of 50% acetic acid aqueous and drying to get a white product, i.e., the 2-methyl-5-iodobenzoic acid of formula II A.

7. The method as claimed in claim 1, **characterized in that**, the base in step 3) is lithium hydroxide, sodium hydroxide or potassium hydroxide.

8. The method as claimed in claim 1, **characterized in that**, the solvent in step 3) is water, or a water-miscible organic solvent.

9. The method as claimed in claim 8, **characterized in that**, the organic solvent is alcoholic solvent or ether solvent.

10. The method as claimed in claim 9, **characterized in that**, the alcoholic solvent is methanol or ethanol, and the ether solvent is THF.

## Patentansprüche

1. Verfahren zum Herstellen von 2-Methyl-5-iod-benzoesäure, umfassend die Schritte von:
1) Verwenden von o-Methyl-benzoesäure der Formel I als Ausgangsmaterial und Jodieren über Iod/Kalium-Persulfat in einem Mischsäurelösungsmittel, um eine Mischung von 2-Methyl-5-iod-benzoesäure der Formel II A, 2-Methyl-3-iod-benzoesäure der Formel II B und 2-Methyl-3,5-diiod-benzoesäure der Formel II C zu erhalten; 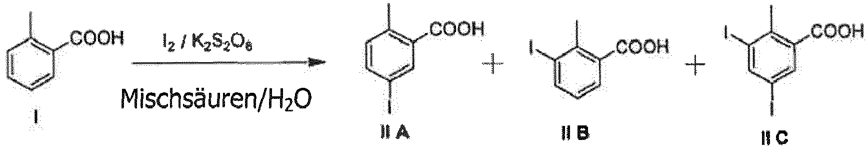
2) Aufbereiten der Mischung, erhaltenen in Schritt 1), durch Umkristallisieren, um 2-Methyl-5-iod-benzoesäure der Formel II A und wiedergewinnbare Mutterlauge zu erhalten;
3) Behandeln und katalytisches Hydrieren der wiedergewinnbaren Mutterlauge in einem Base/Lösungsmittel-System, um o-Methyl-benzoesäure der Formel I und Iodid-Ionen zu erhalten, und dann Oxidieren der Iodid-Ionen in der Gegenwart einer Säure, um Iod wieder zu gewinnen und um die o-Methyl-benzoesäure der Formel I in die Reaktion zum Wiederverwenden zugeben

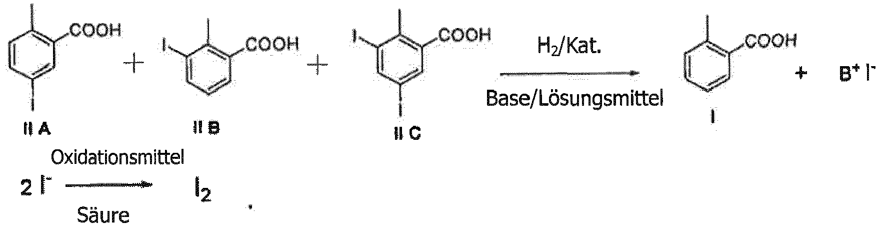

2. Das Verfahren wie in Anspruch 1 beansprucht, **dadurch gekennzeichnet, dass** die Mischsäure in Schritt 1) eine gemischte Lösung von zwei oder mehr von Essigssäure, Propionsäure, Buttersäure und Schwefelsäure in jeglichem Anteil ist.

3. Das Verfahren wie in Anspruch 1 beansprucht, **dadurch gekennzeichnet, dass** Schritt 2) das Aufbereiten der Mischung in einem gemischten Lösungsmittel aus Säure/Wasser umfasst, um 2-Methyl-5-iod-benzoesäure zu erhalten.

4. Das Verfahren wie in Anspruch 3 beansprucht, **dadurch gekennzeichnet, dass** die Säure eine beliebige von Essigsäure, Propionsäure, Buttersäure und Schwefelsäure ist.

5. Das Verfahren wie in Anspruch 1 beansprucht, **dadurch gekennzeichnet, dass** die wiedergewinnbare Mutterlauge in Schritt 2) eine Mischung aus o-Methyl-benzoesäure, 2-Methyl-5-iod-benzoesäure, 2-Methyl-3-iod-benzoesäure und 2-Methyl-3,5-diiod-benzoesäure ist.

6. Das Verfahren wie in Anspruch 1 beansprucht, dadurch gekenneichnet, dass das Aufbereiten in Schritt 2) umfasst: Zugeben von 70% Essigsäure/Wasser in die Mischung erhalten in Schritt 1), Erwärmen bis die Lösung klar wird, langsames Kühlen auf 15∼20°C, Saugfiltrieren, Waschen des Feststoffs mit einer kleinen Menge von 50% wässriger Essigsäure und Trocknen, um ein weißes Produkt zu erhalten, d.h. die 2-Methyl-5-iod-benzoesäure der Formel II A.

7. Das Verfahren wie in Anspruch 1 beansprucht, **dadurch gekennzeichnet, dass** die Base in Schritt 3) Lithiumhydroxid, Natriumhydroxid, oder Kaliumhydroxid ist.

8. Das Vefahren wie in Anspruch 1 beansprucht, **dadurch gekennzeichnet, dass** das Lösungsmittel in Schritt 3) Wasser oder ein Wasser-mischbares organisches Lösungsmittel ist.

9. Das Vefahren wie in Anspruch 8 beansprucht, **dadurch gekennzeichnet, dass** das organische Lösungsmittel ein alkoholisches Lösungsmittel oder Ether-Lösungsmittel ist.

10. Das Verfahren wie in Anspruch 9 beansprucht, **dadurch gekennzeichnet, dass** das alkoholische Lösungsmittel Methanol oder Ethanol ist, und das Ether-Lösungsmittel THF ist.

## Revendications

1. Procédé de préparation d'acide 2-méthyl-5-iodobenzoïque comprenant les étapes de :
1) utilisation de l'acide o-méthylbenzoïque de formule I comme matière première et iodation par l'intermédiaire d'iode/persulfate de potassium dans un solvant acide mixte pour obtenir un mélange d'acide 2-méthyl-5-iodobenzoïque de formule II A, d'acide 2-méthyl-3-iodobenzoïque de formule II B et d'acide 2-méthyl-3,5-diiodobenzoïque de formule II C ; 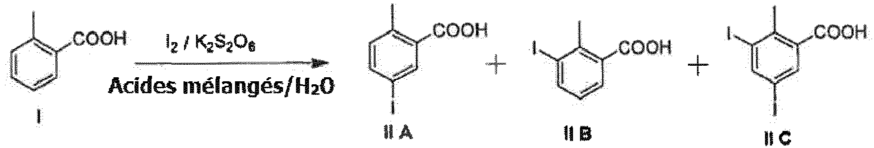
2) raffinage du mélange obtenu à l'étape 1) par recristallisation pour obtenir l'acide 2-méthyl-5-iodobenzoïque de formule II A et une liqueur mère récupérable ;
3) traitement et hydrogénation catalytique de la liqueur mère récupérable dans un système de base/solvant pour obtenir l'acide o-méthylbenzoïque de formule I et des ions iodure, puis oxydation des ions iodure en présence d'un acide pour récupérer l'iode et mettre l'acide o-méthylbenzoïque de formule I dans la réaction pour une réutilisation.

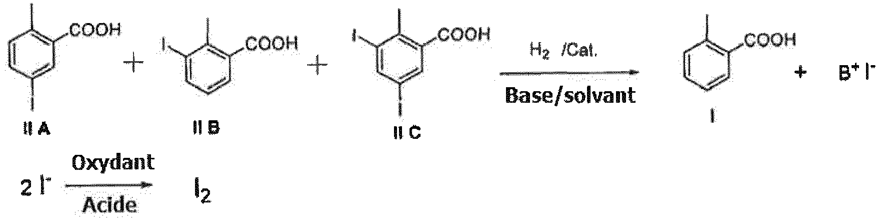

2. Procédé selon la revendication 1, **caractérisé en ce que**, l'acide mixte à l'étape 1) est une solution mixte de plus de deux acides parmi l'acide acétique, l'acide propionique, l'acide butyrique et l'acide sulfurique en n'importe quelle proportion.

3. Procédé selon la revendication 1, **caractérisé en ce que**, l'étape 2) comprend le raffinage du mélange dans un solvant mixte d'acide/eau pour obtenir l'acide 2-méthyl-5-iodobenzoïque.

4. Procédé selon la revendication 3, **caractérisé en ce que**, l'acide est l'un quelconque parmi l'acide acétique, l'acide propionique, l'acide butyrique et l'acide sulfurique.

5. Procédé selon la revendication 1, **caractérisé en ce que**, la liqueur mère récupérable à l'étape 2) est un mélange d'acide o-méthylbenzoïque, d'acide 2-méthyl-5-iodobenzoïque, d'acide 2-méthyl-3-iodobenzoïque et d'acide 2-méthyl-3,5-diiodobenzoïque.

6. Procédé selon la revendication 1, **caractérisé en ce que**, le raffinage à l'étape 2) consiste : à ajouter 70 % d'acide acétique/eau dans le mélange obtenu à l'étape 1), à chauffer jusqu'à ce que la solution devienne limpide, à refroidir lentement jusqu'à 15∼20 °C, à filtrer par aspiration, à laver la matière solide avec une petite quantité d'acide acétique aqueux à 5 % et à sécher pour obtenir un produit blanc, c'est-à-dire, l'acide 2-méthyl-5-iodobenzoïque de formule II A.

7. Procédé selon la revendication 1, **caractérisé en ce que**, la base à l'étape 3) est l'hydroxyde de lithium, l'hydroxyde de sodium ou l'hydroxyde de potassium.

8. Procédé selon la revendication 1, **caractérisé en ce que**, le solvant à l'étape 3) est l'eau ou un solvant organique miscible à l'eau.

9. Procédé selon la revendication 8, **caractérisé en ce que**, le solvant organique est un solvant alcoolique ou un solvant de type éther.

10. Procédé selon la revendication 9, **caractérisé en ce que**, le solvant alcoolique est le méthanol ou l'éthanol, et le solvant de type éther est le THF.
